Europäisches Patentamt

European Patent Office

Office européen des brevets

(19)

(11) Veröffentlichungsnummer: **0 091 499**
**B1**

## (12) EUROPÄISCHE PATENTSCHRIFT

(45) Veröffentlichungstag der Patentschrift:
**06.08.86**

(21) Anmeldenummer: **82103088.9**

(22) Anmeldetag: **10.04.82**

(51) Int. Cl.⁴: **A 61 B 17/58**

(54) **Profilierter intramedullärer Unterschenkelnagel.**

(43) Veröffentlichungstag der Anmeldung:
**19.10.83 Patentblatt 83/42**

(45) Bekanntmachung des Hinweises auf die Patenterteilung:
**06.08.86 Patentblatt 86/32**

(84) Benannte Vertragsstaaten:
**BE CH DE FR GB IT LI NL SE**

(56) Entgegenhaltungen:
**EP - A - 0 008 758
BE - A - 460 027
DE - A - 2 341 439
US - A - 3 977 398**

(73) Patentinhaber: **METALLWERK PLANSEE
GESELLSCHAFT M.B.H., A-6600 Reutte, Tirol (AT)**
Patentinhaber: **Otte, Wolf-Dieter, Am Model 14,
D-8217 Volkach (DE)**

(72) Erfinder: **Otte, Wolf-Dieter, Am Model 14,
D-8712 Volkach (DE)**
Erfinder: **Otte, Heinz, Dr. med., Am Model 14,
D-8712 Volkach (DE)**
Erfinder: **Schider, Siegfried, Dr.,
Mittenwaldbahnstrasse 12, A-6600 Reutte Tirol (AT)**

(74) Vertreter: **Lohnert, Wolfgang, Dr., Metallwerk Plansee
GmbH, A-6600 Reutte, Tirol (AT)**

## Beschreibung

Die Erfindung betrifft einen profilierten, einstückigen, gekrümmten, intramedullären Unterschenkelnagel, welcher dazu bestimmt ist, in einer von der normalen auf die Knochenoberfläche stark abweichenden Richtung in einen Knochen-Einschlagkanal eingebracht zu werden und welcher Elemente für das Ansetzen eines Einschlagwerkzeuges an einem Nagelende aufweist.

Zur Nagelung von Röhrenknochen, bei denen der Knochenmarknagel ausserhalb der Gelenkfläche unter einem spitzen Winkel zur Knochen-Längsachse in einen Knochen-Einschlagkanal eingebracht werden muss, wie das beispielsweise bei der Nagelung von Unterschenkelknochen der Fall ist, werden derzeit in der Knochenchirurgie hauptsächlich genormte, geschlitzte, rohrförmige, mit einem Mantelprofil versehene und gekrümmte Knochenmarknägel verwendet. Ein Endbereich dieser Nägel ist verjüngt und dient als Führung beim Einschlagen. Der andere Endbereich weist eine Stirnfläche senkrecht zur Nagelachse auf und dient zum Ansetzen des Werkzeuges beim Einbringen in den Knochen. Die äussere Mantelfläche des Nagels besitzt ein Längsprofil, das zum Einschlagende hin stetig verschwindet. Die Nägel haben am Einschlagende ringförmigen Querschnitt.

Für den Heilungserfolg bei einer intramedullären Nagelung ist entscheidend, dass der profilierte Knochenmarknagel verdrehsicher im Knochen verankert ist. Eine wesentliche Voraussetzung hierfür ist, dass sich der eingeschlagene Knochenmarknagel zuverlässig rundum gegen die Cortikalis an der Einschlagstelle, das heisst gegen die Mantelfläche des Knochen-Einschlagkanales, abstützen kann. Dabei muss sich der Nagel sowohl gegen den Teil der Mantelfläche, welcher vom eingeschlagenen Nagel aus gesehen gegen die Knochenoberfläche weist als auch gegen den Teil, der dem inneren Knochenmarkkanal benachbart ist, ausreichend abstützen können. Besitzt der Nagel zudem ein geeignetes Mantel-Längsprofil, so ist eine zuverlässige Drehstabilität gewährleistet, ohne dass die für den Heilungserfolg erwünschte Gleitmöglichkeit zwischen Nagel und Knochen in Längsrichtung gänzlich unterbunden wird.

Bekannte gekrümmte intramedulläre Nägel mit einer einschlagseitigen Stirnfläche senkrecht zur Nagelachse, haben im Falle der Einführung in einen schräg zur Knochenoberfläche eingebrachten Einschlagkanal einen grundsätzlichen Nachteil. Entweder werden sie nur so tief eingeschlagen, dass noch eine zuverlässige Abstützung ihres Einschlagendes gegen die Cortikalis des gesamten Knochen-Einschlagkanales gewährleistet ist. Dann ragt aber zwangsläufig das Ende des Nagels über die Knochenoberfläche hinaus und hat Berührung mit dem über dem Knochen liegenden Bindegewebe. Die Folge sind Schmerzen, Gewebeentzündung und ähnliche Komplikationen beim Patienten. Wird demgegenüber der Knochenmarknagel so tief eingeschlagen, dass er nicht mehr über die Knochenoberfläche hinausragt, so hat er im dem Markkanal zugewandten Mantelbereich des Einschlagkanales keine ausreichend grosse Anlagefläche mehr, um die Verdrehsicherheit auf Grund seines Mantelprofils, soweit im Nagel-Endbereich überhaupt noch vorhanden, zuverlässig zu gewährleisten. In ungünstigen Fällen kann der Nagel sogar durch die Cortikalis in den Markkanal hineinbrechen. Er besitzt dadurch keinerlei Drehstabilität mehr und versagt die für eine Heilung erforderliche Fixierung der gebrochenen Knochenteile. Davon abgesehen kann ein vollständig in den Markkanal hineingelangter Knochenmarknagel nur unter Schwierigkeiten und unter Entstehen eines grossen Knochendefektes extrahiert werden.

Es sind Typen von Knochenmarknägeln bekannt, die nicht unmittelbar durch Aufschlagen auf eine stirnseitige Fläche in den Knochen-Einschlagkanal eingebracht werden, sondern die an ihrem einschlagseitigen Ende ein Innengewinde besitzen, in das eine Schlaghülse eingeschraubt wird. Doch auch derartige Nägel haben in der Praxis stirnseitig eine Fläche senkrecht zur Nagelachse und zudem in der Regel keine profilierte Mantelfläche am Einschlagende, so dass für diese beim Einbringen in einen schrägliegenden Knochen-Einschlagkanal die oben geschilderten Nachteile ebenfalls zutreffen.

Es sind auch Nageltypen bekannt, deren einschlagseitiges Ende näherungsweise die Gestalt eines Rundkopfes aufweist. Auch für derartige Nägel treten in etwas abgeschwächter Form die genannten Nachteile auf.

Die GB-PS 817 525 beschreibt einen rotationsstabilen, intramedullären zweiteiligen Nagel, der nach der Fixierung im Knochen, bedingt durch eine unterschiedliche Einschlagtiefe der einzelnen Nagelteile, eine stirnseitig gegliederte Oberfläche am Einschlagende aufweist. Da die beiden Nagelteile nacheinander eingeschlagen werden, wirkt das Einschlagwerkzeug nicht mit dieser gegliederten Oberfläche, sondern jeweils nur mit der ungegliederten Oberfläche jedes einzelnen Nagelteiles zusammen. Ein Überstehen von grösseren Nagelabschnitten über die Knochenoberfläche hinaus ist bei dieser Ausgestaltung eines Knochenmarknagels praktisch nicht zu vermeiden.

Aus der BE-A 460 027 ist es bekannt, die stirnseitige Fläche eines Knochennagels derart abzuschrägen, dass sie bei eingeschlagenem Nagel fluchtend an die Oberfläche des Knochens grenzt. Bei diesem Knochennagel handelt es sich jedoch um einen geraden Schenkelhalsnagel mit verdrehbarem Kopf, der in homogenes, keinen Hohlraum aufweisendes Knochengewebe eingeschlagen wird. Somit liegen bei diesem Knochennagel völlig andere Verhältnisse vor, wie bei einem einstückigen, intramedullären, gekrümmten Unterschenkelnagel, der in den Knochenmarkkanal eingeschlagen wird.

Die EP-A 0 008 758 beschreibt einen Knochenmarknagel, der zur Verbesserung der Verdrehungsfestigkeit im Knochenmarkkanal mit einer speziellen Längsprofilierung versehen ist, die bis zum Einschlagende hin ausgeführt sein kann. Bei dieser Art von Knochennagel handelt es sich jedoch um einen gerade ausgeführten Knochenmarknagel, der gerade in den Markkanal eingeschlagen wird. Die Profilierung bei diesen Knochennägeln bis zum Einschlagende hin vorzusehen, ist aus medizinischer Sicht nur von untergeordneter Bedeutung.

Die Aufgabe vorliegender Erfindung besteht darin, die eingangs geschilderten Nachteile für intramedulläre Unterschenkelnägel zu beseitigen, die unter von der Senkrechten stark abweichender Richtung in einen Knochen-Einschlagkanal eingebracht werden sollen. Die intramedullären Unterschenkelnägel sollen im Einschlagbereich am gesamten Knochengewebe rundum drehstabil anliegen, ohne über die Knochenoberfläche hinauszuragen und die genannten Komplikationen beim Patienten hervorzurufen.

Diese Aufgabe wird erfindungsgemäss dadurch gelöst, dass die mit dem Einschlagwerkzeug zusammenwirkende, stirnseitige Fläche bzw. die stirnseitige Begrenzungsebene einer gegliederten, stirnseitigen Oberfläche des Nagel-Einschlagendes einen spitzen Winkel α mit der Nagel-Längsachse einschliessen, wobei der Nagel in bekannter Weise bis zum Einschlagende hin mit einem Längsprofil versehen ist.

In einer bevorzugten Ausführung ist die stirnseitige Oberfläche am Einschlagende des intramedullären Unterschenkelnagels stufenförmig ausgebildet, wobei mindestens eine Teilfläche senkrecht zur Nagelachse ausgerichtet ist.

In einer weiteren bevorzugten Ausführung weist der intramedulläre Unterschenkelnagel eine schlitzförmige Ausnehmung in Richtung Nagel-Längsachse auf, welche an der stirnseitigen Fläche beginnt.

In einer weiteren bevorzugten Ausführung hat die stirnseitige Fläche in stirnseitiger Ansicht profilierte Ringform mit in etwa gleichbleibender Wandstärke.

Einzelne Ausführungsformen des intramedullären Unterschenkelnagels gemäss der Erfindung werden an Hand der Figuren näher erläutert.

Fig. 1 zeigt in der Seitenansicht einen Unterschenkelnagel mit stufenförmiger Ausgestaltung des einschlagseitigen Endes sowie den hierfür angepassten Teil eines Einschlagwerkzeuges.

Fig. 2 zeigt in der Seitenansicht das einschlagseitige Ende eines Unterschenkelnagels mit geschlitzter Anschlagfläche.

Fig. 3 zeigt das einschlagseitige Ende eines Knochennagels mit Innengewinde, seitlich im Schnitt.

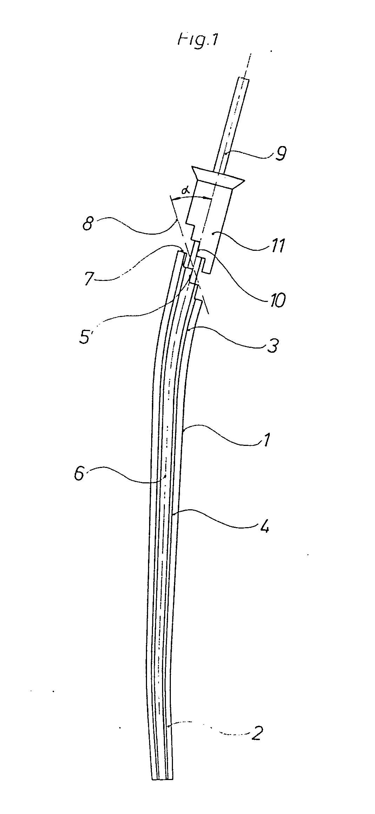

Fig. 1 zeigt einen rohrförmigen, gekrümmten intramedullären Unterschenkelnagel (1), aus einem Material und mit einer eine gute Verdrehsicherheit gewährleistenden Profilierung der Mantelfläche entsprechend der EP-AI 0 008 758. Die kurzen Abschnitte am verjüngten Ende (2) und am einschlagseitigen Ende (3) des Knochennagels sind gegen den vergleichsweise langen Mittelteil (4) geringfügig abgewinkelt. Die Begrenzungsebene (8) der stirnseitig gegliederten Fläche (5) am Nagel-Einschlagende (3) schliesst mit der Längsachse (6) des Knochennagels einen spitzen Winkel α ein. Die Neigung ist dabei so gewählt, dass die Begrenzungsebene (8) bei vollständig eingeschlagenem Knochennagel in etwa fluchtend mit der Knochenoberfläche verläuft. Die stirnseitige Fläche (5) zeigt Stufenform mit senkrecht zur Nagel-Längsachse (6) stehenden Teilflächen (7). Das Einschlagen des Knochennagels erfolgt beispielsweise mit Hilfe des abgebildeten Einschlagwerkzeuges. Der Führungsteil (9) ist in das Ende des Knochenmarknagels eingeschraubt. Auf den Führungsteil ist eine Einschlaghülse (11) aufgesteckt, deren Endfläche (10) der stirnseitigen Fläche (5) des Knochenmarknagels angepasst ist. Als Schlaghammer dient beispielsweise eine gleitend auf den Führungsteil aufgesteckte, schwere Schlagbüchse.

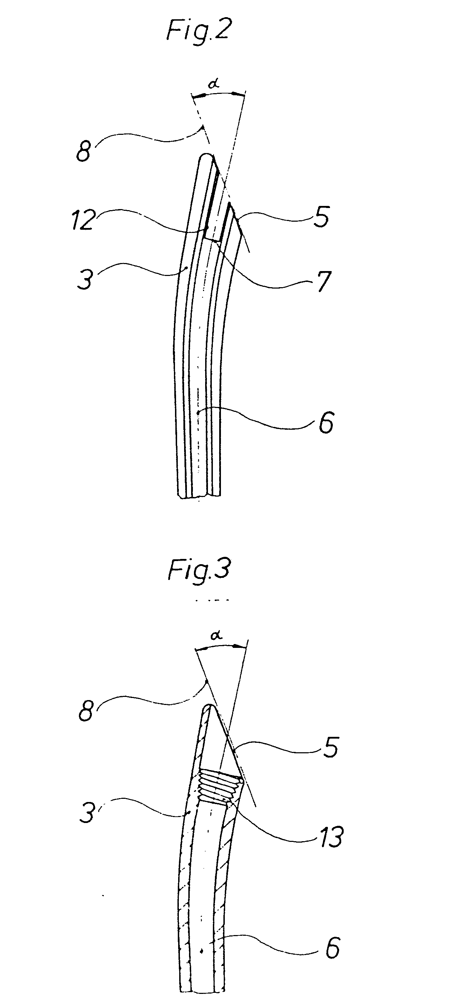

Fig. 2 zeigt in Seitenansicht das Nagel-Einschlagende (3), bei dem die stirnseitige Fläche (5) durch eine schlitzförmige Ausnehmung (12) unterteilt ist. Die Teilfläche (7) dieser Ausnehmung verläuft senkrecht zur Nagel-Längsachse (6).

Fig. 3 zeigt den Einschlagbereich eines weiteren Knochennagels gemäss der Erfindung im Schnitt von der Seite. Der Knochennagel hat Rohrform. Im an die stirnseitige Fläche (5) zur Nagelmitte hin angrenzenden Nagelbereich ist ein Innengewinde (13) angebracht. Das Gewinde ist in der dargestellten Ausführung konisch, sich zur Nagelmitte hin verjüngend. In dieses wird zum Einschlagen des Nagels eine Einschlaghülse eingeschraubt, die ihrerseits am gegenüberliegenden Ende eine Aufschlagfläche senkrecht zur Nagel-Längsachse besitzt.

Allen diesen Nagelausführungen ist gemeinsam, dass sie beim Einführen in einen schrägliegenden Knochen-Einschlagkanal mit der profilierten Mantelfläche rundum und damit verdrehsicher an der Cortikalis anliegen, ohne über die Knochenoberfläche vorzustehen. Dieser Vorteil wirkt sich in keinem Fall nachteilig auf die Handhabung, insbesondere das Einschlagen in und das Ausziehen aus dem Knochen aus. Komplikationen der eingangs geschilderten Art können beim Patienten hierdurch vermieden werden.

Der erfindungsgemässe Unterschenkelnagel ist nicht auf die beschriebenen Formen und auf die Verwendung bestimmter Werkstoffe beschränkt.

**Patentansprüche**

1. Profilierter, einstückiger, gekrümmter, intramedullärer Unterschenkelnagel, welcher dazu bestimmt ist, in einer von der Normalen auf die Knochenoberfläche stark abweichenden Richtung in einen Knochen-Einschlagkanal eingebracht zu werden, und welcher Elemente für das Ansetzen eines Einschlagwerkzeuges an einem Nagelende aufweist, dadurch gekennzeichnet, dass die mit dem Einschlagwerkzeug (11) zusammenwirkende, stirnseitige Fläche (5), bzw. die stirnseitige Begrenzungsebene (8) einer gegliederten, stirnseitigen Oberfläche des Nagel-Einschlagendes (3), einen spitzen Winkel ($\alpha$) mit der Nagel-Längsachse (6) einschliesst, wobei der Nagel in bekannter Weise bis zum Einschlagende hin mit einem Längsprofil versehen ist.

2. Profilierter intramedullärer Unterschenkelnagel nach Anspruch 1, mit einer stirnseitigen gegliederten Oberfläche, dadurch gekennzeichnet, dass die stirnseitige gegliederte Oberfläche (5) mindestens eine Teilfläche (7) senkrecht zur Nagel-Längsachse (6) besitzt.

3. Profilierter intramedullärer Unterschenkelnagel nach Anspruch 1 oder 2, mit einer stirnseitigen gegliederten Oberfläche, dadurch gekennzeichnet, dass die stirnseitige gegliederte Oberfläche (5) mehrere Stufen besitzt.

4. Profilierter intramedullärer Unterschenkelnagel nach Anspruch 1, mit einer stirnseitigen Fläche (5), dadurch gekennzeichnet, dass an der stirnseitigen Fläche (5) eine schlitzförmige Ausnehmung (10) in Richtung Nagel-Längsachse (6) ansetzt.

5. Profilierter intramedullärer Unterschenkelnagel nach einem der Ansprüchen 1 oder 4, mit einer stirnseitigen Fläche (5), dadurch gekennzeichnet, dass die stirnseitige Fläche (5) in stirnseitiger Ansicht, eine profilierte Ringform mit in etwa gleichbleibender Wandstärke aufweist.

## Claims

1. Profilated unitary curved intramedullary lower leg spike suitable for being driven into a spike receiving duct in the bone in a direction deviating greatly from a normal line onto the bone surface, said spike comprising elements for applying a driving tool to one end of the spike, characterized in that the face area (5) engaged with the driving tool (11) and the face plane (8) respectively, bordering the structured face surface of the striking end (3) of the spike, form an acute angle ($\alpha$) with the longitudinal axis (6) of the spike, said spike having in known manner a profile in longitudinal direction up to the striking end.

2. Profilated intramedullary lower leg spike according to claim 1 with a structured face surface, characterized in that the structured face surface (5) comprises at least one partial area (7) perpendicular to the longitudinal axis (6) of the spike.

3. Profilated intramedullary lower leg spike according to claim 1 or 2 with a structured face surface, characterized in that the structured face surface (5) comprises a plurality of steps.

4. Profilated intramedullary lower leg spike according to claim 1 with a face area (5), characterized in that a slot shaped recess (10) is applied at the face area (5) having its direction equally to the longitudinal axis (6) of the spike.

5. Profilated intramedullary lower leg spike according to one of the claims 1 or 4 with a face area (5), characterized in that the face area (5), viewed in a longitudinal direction, has a profilated annular shape with walls of substantially uniform thickness.

## Revendications

1. Clou intramédullaire pour jambe, profilé, courbé et d'un seul tenant, destiné à être agencé dans un canal d'enfoncement dans un os, dans une direction différant fortement de la normale à la surface de l'os, ledit clou présentant des éléments pour l'application d'un outil d'enfoncement à l'une de ses extrémités, caractérisé en ce que la surface de côté frontal (5) coopérant avec l'outil d'enfoncement (11), ou encore le plan (8) de délimitation d'une surface de côté frontal segmentée (5) de l'extrémité enfoncement (3) du clou, forme un angle aigu ($\alpha$) avec l'axe longitudinal (6) du clou, ledit clou étant muni, de façon connue, d'un profil longitudinal jusqu'à l'extrémité enfoncement.

2. Clou intramédullaire profilé pour jambe, selon la revendication 1, avec une surface de côté frontal segmentée, caractérisé en ce que ladite surface de côté frontal segmentée (5) possède au moins une surface partielle (7) perpendiculaire à l'axe longitudinal (6) du clou.

3. Clou intramédullaire profilé pour jambe, selon la revendication 1 ou 2, avec une surface de côté frontal segmentée, carctérisé en ce que ladite surface de côté frontal segmentée (5) possède plusieurs gradins.

4. Clou intramédullaire profilé pour jambe, selon la revendication 1, avec une surface de côté frontal (5), caractérisé en ce qu'un évidement (10) en forme de fente fait suite à la surface de côté frontal (5), dans la direction de l'axe longitudinal (6) du clou.

5. Clou intramédullaire profilé pour jambe, selon l'une des revendications 1 ou 4, avec une surface de côté frontal (5), caractérisé en ce que ladite surface de côté frontal (5) présente, quant on la considère par le côté frontal, une forme annulaire profilée avec une épaisseur de paroi sensiblement constante.

Fig.1

## Fig.2

## Fig.3